# EUROPÄISCHE PATENTSCHRIFT

(11) **EP 2 111 793 B1**
(45) Veröffentlichungstag und Bekanntmachung des Hinweises auf die Patenterteilung: **25.02.2015**
(21) Anmeldenummer: 08169933.2
(22) Anmeldetag: 10.01.2004
(51) Int. Cl.: A61B 5/15

(54) **Vorrichtung und Verfahren zur Aufnahme einer Körperflüssigkeit für Analysezwecke**
Device and method for containing a bodily fluid for analysis purposes
Dispositif et procédé destinés à la réception d'un liquide corporel à des fins d'analyse

(30) Priorität: 23.01.2003 DE 10302501
(43) Veröffentlichungstag der Anmeldung: 28.10.2009
(62) Teilanmeldung aus: 04701300.8
(73) Patentinhaber: F. Hoffmann-La Roche AG, 4070 Basel (CH); Roche Diagnostics GmbH, 68305 Mannheim (DE)
(72) Erfinder: Sacherer, Klaus-Dieter, 67281, Kirchheim (DE); Mönch, Ronald, 68549, Ilvesheim (DE); Schabbach, Michael, 69469, Weinheim (DE); Scherer, Jörg, 4258, Zuchwill (CH)
(74) Vertreter: Pfiz, Thomas

(56) Entgegenhaltungen:
- EP-A- 0 301 165
- WO-A-02/100276
- WO-A2-02/100251
- GB-A- 2 335 990
- US-A1- 2002 177 787
- US-B1- 6 306 152
- US-B1- 6 332 871

## Beschreibung

Die Erfindung betrifft eine Vorrichtung zur Aufnahme einer Körperflüssigkeit für Analysezwecke gemäß dem Oberbegriff der unabhängigen Patentansprüche.

Für die Patienten-Selbstkontrolle insbesondere bei Diabeteserkrankungen sind Einmal- bzw. Schnelltests bekannt, bei denen ein analytisches Testelement mit geringen Mengen einer Körperflüssigkeit beaufschlagt wird, um in einem automatischen Messablauf eine Stoffwechsetgröße zu bestimmen. Speziell für die Blutglucosebestimmung wurden federgetriebene Stechhilfen zur Gewinnung von Kapillarblut entwickelt, welche vom Benutzer beispielsweise an der Fingerbeere angesetzt werden, um durch einen möglichst schmerzarmen Einstich eine ausreichende Blutmenge für die nachfolgende Analyse zu gewinnen. Hierbei wird das ausgetretene Blut mit einem aus einem Messgerät ausgestoßenen Teststreifen als Probenaufnahmeeinheit aufgetupft und dieser nach erfolgter Messung als Verbrauchsmittel verworfen. Neben den immer noch zeitaufwändigen Detailschritten liegt ein besonderes Problem auf diesem Gebiet in der Kontaminations- bzw. Infektionsgefahr durch unkontrolliert in die Umwelt abgegebene Verbrauchsmittel.

Eine Vorrichtung gemäß der Präambel von Anspruch 1 ist aus der US 2002/177787 bekannt.

Ausgehend hiervon liegt der Erfindung die Aufgabe zugrunde, die im Stand der Technik aufgetretenen Nachteile zu vermeiden und ein System bzw. Verfahren der eingangs angegebenen Art zu optimieren, so dass eine einfache und weitgehend situationsunabhängige Bedienung auch für Laien möglich ist und eine besonders hygienische Handhabung sichergestellt wird.

Zur Lösung dieser Aufgabe wird die in den unabhängigen Patentansprüchen jeweils angegebene Merkmalskombination vorgeschlagen. Vorteilhafte Ausgestaltungen und Weiterbildungen der Erfindung ergeben sich aus den abhängigen Ansprüchen.

Die Erfindung basiert auf dem Gedanken, eine Probenaufnahmeeinheit als solche anzutreiben und hierfür mit geeigneten Elementen auszurüsten. Dementsprechend wird erfindungsgemäß eine Kopplungsvorrichtung zum Koppeln der Probenaufnahmeeinheit mit der Antriebseinheit für eine Hin- und Rückbewegung zwischen der Führungskammer und der Aufnahmestelle vorgeschlagen. Der Benutzer muss also die Probenaufnahmeeinheit nicht berühren, sondern kann dies durch das System automatisch ausführen lassen. Dadurch lässt sich die Messung beschleunigt und mit einem hohen Maß an Zuverlässigkeit und Sicherheit durchführen, und es kann durch die Rekassettierung bzw. Rückführung in das Behältnis eine hygienische Handhabung und Entsorgung sichergestellt werden. Für den Patienten bedeutet dies eine erhebliche Erleichterung nicht zuletzt durch die Möglichkeit einer diskreteren Benutzung, ohne sogleich beispielsweise als Diabetiker erkannt zu werden.

Eine weitere Verbesserung in dieser Hinsicht wird dadurch erreicht, dass die Kopplungsvorrichtung vorzugsweise selbsttätig arbeitende Verbindungsmittel zum Herstellen und Lösen einer Formschlussverbindung zwischen Antriebseinheit und Probenaufnahmeeinheit aufweist, wobei die Antriebseinheit und die Probenaufnahmeeinheit in einer Ausgangsstellung voneinander getrennt sind. Dies lässt sich vorteilhafterweise dadurch realisieren, dass die Kopplungsvorrichtung mindestens einen bei der Hin- und Rückbewegung wegabhängig zwischen einer Freigabestellung und einer Eingriffstellung bewegbaren Mitnehmer zur Kopplung von Antriebseinheit und Probenaufnahmeeinheit aufweist. Für einen selbstgesteuerten Ablauf ist es von Vorteil, wenn die Kopplungsvorrichtung eine bei der Hin- und Rückbewegung durch den Mitnehmer abtastbare, insbesondere durch eine Anlaufschräge der Führungskammer gebildete Steuerkulisse aufweist.
Eine Vorrichtung gemäss der Erfindung ist in Anspruch 1 definiert.

Vorteilhafterweise ist der Mitnehmer an einem proximalen Ende der Probenaufnahmeeinheit angeordnet und durch mindestens eine vorzugsweise unter Eigenspannung in eine Eingriffstellung einrückbare Halteklaue gebildet. Eine mechanisch besonders einfache Ausführung sieht vor, dass die Antriebseinheit einen Stößel aufweist, und dass der Mitnehmer bei einem Axialvorschub des Stößels selbsttätig mit einer Kopfpartie des Stößels in Eingriff kommt.

Eine besonderer Aspekt der Erfindung besteht in einem an der Probenaufnahmeeinheit einhakbaren Hakenstößel als Kopplungsvorrichtung. Für die Hin- bzw. Vorschubbewegung sieht eine vorteilhafte Ausgestaltung vor, dass der Hakenstößel eine gegen die Probenaufnahmeeinheit anschlagende Schubflanke aufweist. Zum Rückholen der Probenaufnahmeeinheit ist es vorteilhaft, wenn der Hakenstößel eine mit der Probenaufnahmeeinheit in Eingriff bringbare Zugflanke aufweist. Um das Ein- und Ausklinken zu erleichtern, ist es günstig, wenn die Zug- und/oder die Schubflanke zu ihrer freien Randkante hin in Richtung der Hinbewegung geneigt sind.

Um eine zuverlässige Kopplung zu erreichen, ist es von Vorteil, wenn der Hakenstößel einen abgekröpften Hakenkopf aufweist, wobei der Hakenkopf bei der Kopplung zu der Probenaufnahmeeinheit hin seitlich vorspringt. Eine weitere Verbesserung des ergibt sich dadurch, dass der Hakenstößel über ein bei der Hin- und Rückbewegung schräg gegen eine Führungskontur anlaufendes Stößelstück gegenüber der Probenaufnahmeeinheit verschwenkbar ist. Hierbei ist es günstig, wenn der Hakenstößel in einem der Führungskammer vorgelagerten, in Richtung der Hinbewegung sich verjüngenden Führungskonus geführt ist, wobei der Führungskonus gegenüber der Zentralachse der Führungskammer exzentrisch versetzt ist.

Um das Durchstechen einer Schutzfolie und das Einfahren in die Eingriffstellung zu erleichtern, ist es von Vorteil, wenn der Hakenstößel einen stirnseitig angeformten, gegen die Führungskammer weisenden Dornfortsatz aufweist.

Um das Einklinken zu erleichtern und nach der Rückholung eine definierte Sicherung der Probenaufnahmeeinheit zu gewährleisten, ist es vorteilhaft, wenn die robenaufnahmeeinheit durch eine in die Führungskammer vorspringende Klemmstruktur lösbar gehalten ist, wobei die Klemmkraft der Klemmstruktur kleiner als die maximale Antriebskraft der Antriebseinheit sein sollte. Dabei ist es für eine raumsparende Bauform vorteilhaft, wenn die Probenaufnahmeeinheit einen unter Freigabe eines Durchtrittsquerschnitts der Führungskammer zum Ein- und/oder Ausklinken des Hakenstößels in der Klemmstruktur elastisch verformten proximalen Endabschnitt aufweist. Um dies zu erreichen, ist es vorteilhaft, wenn die Klemmstruktur zwei parallel zueinander längs der Führungskammer verlaufende Führungsrippen und zwei in einem der Antriebseinheit zugewandten Klemmbereich der Führungskammer angeordnete, vorzugsweise mit Seitenversatz gegen die Führungsrippen vorspringende Klemmnocken aufweist.

Vorteilhafterweise besitzt die Probenaufnahmeeinheit eine Ausnehmung als Kopplungselement zum Einhaken des Hakenstößels.

Gemäss der Erfindung ist in der Probenaufnahmeeinheit eine Stecheinheit zur Ausführung einer Stechbewegung gegen ein die Körperflüssigkeit enthaltendes Körperteil integriert. Der Einstich und die Probenaufnahme können somit in einem automatischen Bewegungsablauf besonders einfach und hygienisch unter Vermeidung jedweder manueller Eingriffe des Benutzers erfolgen.

Für eine besonders günstige teleskopartige Bewegung ist es von Vorteil, wenn die Stecheinheit in einer Führung der Probenaufnahmeeinheit in deren Bewegungsrichtung verschiebbar ist. Dabei kann die Gewinnung der Körperflüssigkeit dadurch optimiert werden, dass die Stecheinheit in einem vorgegebenen Abstand zu einem freien Stirn- bzw. Aufnahmebereich der Probenaufnahmeeinheit in das Körperteil einstechbar ist.

Für eine gesonderte Bewegungssteuerung ist es von Vorteil, wenn die Stecheinheit über einen zugeordneten Mitnehmer der Kopplungsvorrichtung für eine hin- und hergehende Stechbewegung formschlüssig mit der Antriebseinheit koppelbar ist. Eine baulich besonders günstige Ausführung sieht vor, dass die Antriebseinheit einen durch einen Außenstößel und einen darin längsverschiebbaren Innenstößel gebildeten Doppelstößel aufweist.

Zur Ablaufsteuerung der Bewegung von Probenaufnahmeeinheit und Stecheinheit weist die Antriebseinheit eine Steuereinrichtung auf. Zur Steuerung einer Relativbewegung ist es auch möglich, dass die Probenaufnahmeeinheit vorzugsweise durch vorspringende Körperkanten gebildete Anschläge für die Stecheinheit aufweist.

Eine weitere vorteilhafte Ausführung sieht vor, dass die Stecheinheit entgegen der Rückstellkraft eines Federglieds gegenüber der Probenaufnahmeeinheit begrenzt verschieblich ist.

Für einen möglichst energiesparenden Bewegungsablauf ist es vorteilhaft, wenn die Probenaufnahmeeinheit in einer Gleitführung der Führungskammer verschiebbar ist.

Um die Führung und Zentrierung an der vorgesehenen Aufnahmestelle weiter zu verbessern, ist es von Vorteil, wenn die Probenaufnahmeeinheit an einem mittels der Antriebseinheit in der Führungskammer hin und her verschiebbaren Schlitten vorzugsweise über eine Rastverbindung gehalten ist.

Vorteilhafterweise besitzt die Probenaufnahmeeinheit einen vorzugsweise kapillaraktiven Transportkanal für eine möglichst selbsttätige Fließverbindung von der Aufnahmestelle zu einer Auswertestelle, welche durch ein spezielles analytisches Testelement zur Untersuchung der Körperflüssigkeit an der Probenaufnahmeeinheit gebildet sein kann.

Eine weitere vorteilhafte Ausführung sieht vor, dass der Transportkanal durch einen Ringspalt zwischen einer Lanzette und einem die Lanzette umgebenden Wandbereich der Probenaufnahmeeinheit gebildet ist. Um den Flüssigkeitstransport günstig zu unterstützen, ist es vorteilhaft, wenn der ringförmige Transportkanal in einem von der unter Schwerkraft belasteten Auflageseite der Lanzette abgewandten Transportbereich einen erweiterten Querschnitt besitzt.

Hierbei ist es günstig, wenn der Transportkanal über eine seitliche Auslassöffnung vorzugsweise in dem Transportbereich auf einem analytischen Testfeld mündet. Möglich ist es auch, dass der Transportkanal über eine in Kanalrichtung weisende axiale Auslassöffnung an einer analytischen Testhülse mündet.

Zum Schutz vor schädlichen Umwelteinflüssen ist es günstig, wenn die Führungskammer zumindest im Bereich einer Ausschuböffnung durch eine Siegelfolie verschlossen ist. Dabei sollte die Probenaufnahmeeinheit einen in Vorschubrichtung weisenden freien Stirnbereich zum Durchstoßen der Siegelfolie aufweisen, so dass Beschädigungen der Stecheinheit vermieden werden.

Vorteilhafterweise ist die Probenaufnahmeeinheit als Teststreifen oder vorzugsweise spritzgegossener Testkörper insbesondere für Blutuntersuchungen ausgebildet. Eine weitere vorteilhafte Variante sieht vor, dass die Probenaufnahmeeinheit durch eine vorzugsweise zugleich als Führung für die Stecheinheit ausgebildete Kanüle zum Ansaugen der Probenflüssigkeit gebildet ist.

Um den Bedienungskomfort weiter zu verbessern, ist es vorteilhaft, wenn das Behältnis als Magazin zur Bevorratung einer Mehrzahl von Probenaufnahmeeinheiten ausgebildet ist. In einer für die Bewegungssteuerung günstigen Ausführung kann das Behältnis als Trommelmagazin eine Mehrzahl von in Umfangsrichtung verteilt angeordneten, axial verlaufenden Führungskammern für jeweils eine Probenaufnahmeeinheit aufweist. Alternativ sieht eine besonders kompakte Ausführung vor, dass das Behältnis als Scheibenmagazin eine Mehrzahl von sternförmig angeordneten, radial verlaufenden Führungskammern für jeweils eine Probenaufnahmeeinheit aufweist.

Die Erfindung erstreckt sich auch auf ein Analysegerät, insbesondere ein transportables Handgerät für die medizinische Diagnostik, mit einer erfindungsgemäßen Vorrichtung zur Aufnahme einer Körperflüssigkeit.

In verfahrensmäßiger Hinsicht wird die eingangs genannte Aufgabe dadurch gelöst, dass die mit der Antriebseinheit koppelbare Probenaufnahmeeinheit nach der Probenaufnahme in die Führungskammer zurückgezogen wird. Vorteilhafterweise wird die Körperflüssigkeit durch eine Stechbewegung einer in der Probenaufnahmeeinheit verschiebbaren Stecheinheit im Bereich der Aufnahmestelle gewonnen.

Im Folgenden wird die Erfindung anhand der in der Zeichnung in schematischer Weise dargestellten Ausführungsbeispiele näher erläutert. Es zeigen:
- Fig. 1: eine Vorrichtung zur Aufnahme und Analyse von Kapillarblut in einer axial aufgebrochenen perspektivischen Ansicht;
- Fig. 2: eine Probenaufnahmeeinheit der Vorrichtung nach Fig. 1 in einer Ausgangsstellung gegenüber einer Antriebseinheit in einem ausschnittsweisen Axialschnitt;
- Fig. 3 bis 5: verschiedene Vorschubstellungen der mit der Antriebseinheit gekoppelten Probenaufnahmeeinheit zur Blutentnahme aus der Fingerbeere in einer Fig. 2 entsprechenden Darstellung;
- Fig. 6: ein weiteres Ausführungsbeispiel mit einer aus Folienteilen gebildeten streifenförmigen Probenaufnahmeeinheit in einer Fig. 1 entsprechenden Darstellung;
- Fig. 7: die Probenaufnahmeeinheit nach Fig. 6 in einem senkrecht zu der Streifenebene verlaufenden Längsmittelschnitt;
- Fig. 8 bis 10: verschiedene Vorschubpositionen der Probenaufnahmeeinheit in einer Fig. 3 bis 5 entsprechenden Darstellung;
- Fig. 11: ein weiteres Ausführungsbeispiel mit einer an einem Schlitten gelagerten streifenförmigen Probenaufnahmeeinheit in einer Fig. 6 entsprechenden Ansicht;
- Fig. 12: einen ausschnittsweisen Mittelschnitt der Probenaufnahmeeinheit nach Fig. 11 senkrecht zur Streifenebene;
- Fig. 13: ein weiteres Ausführungsbeispiel mit an einem Federglied gelagerter streifenförmiger Probenaufnahmeeinheit in einer aufgebrochenen perspektivischen Ansicht;
- Fig. 14: die Probenaufnahmeeinheit nach Fig. 13 in einer Fig. 12 entsprechenden Darstellung;
- Fig. 15: ein Ausführungsbeispiel einer scheibenförmigen Vorrichtung zur Aufnahme und Untersuchung von Blutproben in einem Axialschnitt;
- Fig. 16 und 17: die Vorrichtung nach Fig. 15 in einer Ausgangsstellung und einer Entnahmestellung einer radial ausschiebbaren Probenaufnahmeeinheit in einer ausschnittsweisen perspektivischen Ansicht;
- Fig. 18: ein weiteres Ausführungsbeispiel mit einem Hakenstößel für eine Vorschub- und Rückholbewegung eines Teststreifens in einem Längsschnitt;
- Fig. 19: eine abgebrochene Seitenansicht eines Kopfstücks des abgekröpften Hakenstößels;
- Fig. 20: einen in einer Führungskammer klemmend gehaltenen Teststreifen in einer Rückseitenansicht;
- Fig. 21: einen Längsschnitt durch die Führungskammer nach Fig. 20; und
- Fig. 22 bis 24: verschiedene Vorschubstellungen des an dem Teststreifen einhakbaren Hakenstößels in einer perspektivischen Ansicht.

Die in der Zeichnung dargestellte Analysevorrichtung dient zur Blutuntersuchung für die Patienten-Selbstkontrolle speziell für Diabetiker. Das System umfasst ein Vorratsbehältnis 10 mit einer Mehrzahl von Führungskammern 12, in den Führungskammern jeweils einzeln angeordnete Probenaufnahmeeinheiten 14 mit gegebenenfalls integrierter Stecheinheit 16 sowie zugehörige Kopplungsvorrichtungen 18 zum Koppeln der Probenaufnahmeeinheiten bzw. Stecheinheiten mit einer Antriebseinheit 20 für eine Hin- und Rückbewegung zwischen der jeweiligen Führungskammer 12 und einer Aufnahmestelle 22 im Bereich eines Körperteils 24 für die Blutgewinnung.

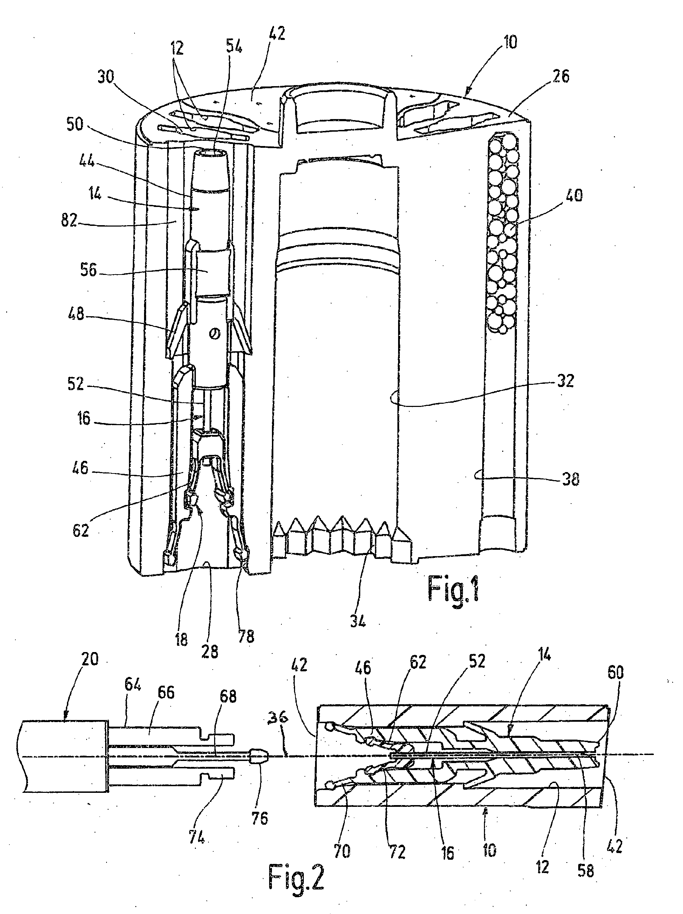

Wie in Fig. 1 gezeigt, ist das Behältnis 10 durch ein als zylindrisches Spritzgussteil aus Kunststoff ausgebildetes Trommelmagazin 26 gebildet. Die Führungskammern 12 sind darin in Umfangsrichtung verteilt angeordnet und verlaufen axial durchgehend zwischen einer stirnseitigen Eingriffsöffnung 28 für die Antriebseinheit 20 und einer gegenüberliegenden Ausschuböffnung 30 für die Probenaufnahmeeinheit 14. Das Trommelmagazin 26 besitzt eine Zentralbohrung 32 mit Randverzahnung 34 für ein nicht gezeigtes Schrittschaltwerk zur fluchtenden Positionierung einer auszustoßenden Probenaufnahmeeinheit 14 in der Vorschubachse 36 der Antriebseinheit 20. Radial nach außen versetzt sind axiale Sacklochbohrungen 38 zur Aufnahme eines Trockenmittels 40 angeordnet. Zum Schutz vor schädlichen Umwelteinflüssen sind die Führungskammern 12 stirnseitig durch eine in Fig. 1 nur ausschnittsweise gezeigte Siegelfolie 42 verschlossen.

Die Probenaufnahmeeinheiten 14 sind als so genanntes "Disposable" für den Einmalgebrauch bestimmt. Bei der in Fig. 1 gezeigten Ausführungsform ist hierfür ein hohlzylindrisches Spritzgussteil 44 aus Kunststoff vorgesehen, an welchem Mitnehmer 46 der Kopplungsvorrichtung 18 sowie Anschlagstücke 48 seitlich abstehend angeformt sind. Ein Axialkanal 50 dient zur längsverschieblichen Lagerung einer Lanzette 52 der Stecheinheit und zugleich zumindest bereichsweise als Transportkanal zum selbsttätigen Kapillartransport der gewonnenen Blutflüssigkeit von einer in Vorschubrichtung weisenden Aufnahmeöffnung 54 zu einem Testfeld 56. Dieses Testfeld 56 ist in an sich bekannter Weise zum Nachweis eines Inhaltsstoffs der gewonnenen Blutprobe, speziell für einen Glukosetest ausgebildet.

Die Lanzettenspitze 58 der Lanzette 52 ist im Ausgangszustand gegenüber dem Stirnrand 60 der Probenaufnahmeeinheit 14 in Vorschubrichtung gesehen zurückversetzt angeordnet. Das proximale Lanzettenende ist mit einem in dem Mitnehmer 42 der Probenaufnahmeeinheit gelagerten zweiten Mitnehmer 62 für eine gesonderte Antriebskopplung versehen.

Entsprechend besitzt die in Fig. 2 gezeigte Antriebseinheit 20 einen Doppelstößel 64 zur Ankopplung der Probenaufnahmeeinheit 14 und der Stecheinheit 16. Dieser ist durch einen Außenstößel 66 und einen darin teleskopierbaren Innenstößel 68 gebildet. Die hin- und hergehende Bewegungsübertragung über erfolgt über eine jeweilige Formschlussverbindung der Mitnehmer 46, 62 der Kopplungsvorrichtung 18, wie sie nachstehend näher erläutert wird.

Die Mitnehmer 46, 62 umfassen jeweils zwei Halteklauen 70, 72, welche unter Eigenspannung zangenartig gegeneinander bewegbar sind, um ein zugeordnetes Kopfstück 74, 76 des Außenstößels 66 bzw. Innenstößels 68 formschlüssig zu hintergreifen. Das Einrücken in die Eingriffstellung geschieht beim Stößelvorschub selbsttätig durch Abfahren einer zugeordneten Steuerkulisse 78, 80, wobei die Kulisse 78 für die außen liegenden Halteklauen 70 durch die mit Anlaufschrägen 77 versehenen Schmalseiten der Führungskammern 12 gebildet ist, während der äußere Mitnehmer 46 an seinen Innenflanken eine entsprechende Kulisse 80 für die Halteklauen 72 des inneren Mitnehmers 62 der Stecheinheit 16 bilden.

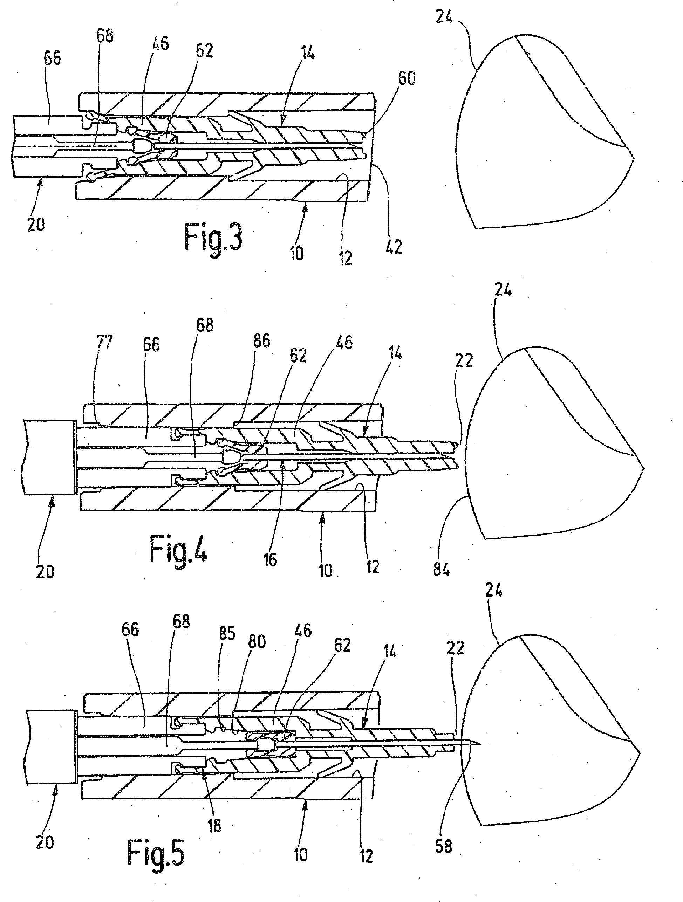

In der in Fig. 2 gezeigten Ausgangsstellung der Antriebseinheit 20 kann das Trommelmagazin 26 revolverartig gedreht werden, um die gewünschte Probenaufnahmeeinheit 14 zur Antriebskopplung zu positionieren. Sodann wird in einem nächsten Schritt gemäß Fig. 3 der Doppelstößel 64 unter Durchstoßen der Siegelfolie 42 über der Eingriffsöffnung 28 in die Führungskammer 12 eingefahren und dabei in stirnseitigen Anschlag gegen die Mitnehmer 46, 62 gebracht. Nach dem Passieren der Anlaufschräge 77 gelangt entsprechend Fig. 4 zunächst der äußere Mitnehmer 46 in seine Eingriffstellung mit dem Außenstößel 66. Die Seitenführung wird dabei durch die Breitseiten 82 der Führungskammer 12 unterstützt, welche als Gleitflächen an die Außenkontur der Probenaufnahmeeinheit 14 angepasst sind. Beim weiteren Vorschub durchstößt der vorspringende freie Stirnrand 60 die Probenaufnahmeeinheit 14 die Siegelfolie 42 über der Ausstoßöffnung 30, wobei die zurückversetzte Lanzettenspitze 58 gegen ungewolltes Umbiegen geschützt bleibt. Wie in Fig. 4 gezeigt, wird die Vorschubbewegung der Probenaufnahmeeinheit 14 an der vorgesehenen Aufnahmestelle 22 in einem vorgegebenen Abstand zu der Fingerbeere 84 gestoppt, um den Blutaustritt beim nachfolgenden Stechvorgang nicht zu behindern.

Gemäß Fig. 5 findet der Stechvorgang bei verschiebefest in seiner Eingriffstellung gehaltenem äußerem Mitnehmer 46 durch weiteren Vorschub des Innenstößels 68 statt, wobei auch der innere Mitnehmer 62 der Stecheinheit 16 nach Passieren der Anlaufschräge 85 in seine Eingriffstellung gelangt. Die Stechtiefe wird durch den zur Verfügung stehenden Verschiebeweg bis zu der gezeigten Anschlaglage des inneren Mitnehmers 62 begrenzt, wobei die Stechgeschwindigkeit für einen schmerzarmen Einstich möglichst hoch sein sollte.

Nach dem Einstich erfolgt die Rückholung der Lanzette 52 und der Probenaufnahmeeinheit 14 in umgekehrter Sequenz entsprechend Fig. 4 bis 2. An der Einstichstelle wird nur eine mikroskopische Menge (Mikroliter) an austretendem Blut benötigt, das über den Kapillarspalt an der Mantelseite der Lanzette 52 durch Kapillarwirkung selbsttätig zu dem Testfeld 56 fließt. Aufgrund der auch in Rückholrichtung bestehenden Formschlussverbindung der Mitnehmer 46, 62 kann die Probenaufnahmeeinheit 14 vollständig in die Führungskammer 12 zurückgezogen werden, bis schließlich die Anschlagstücke 48 gegen die Wandstufe 86 der Führungskammer 12 anschlagen und der Doppelstößel 64 wieder freigegeben wird.

Bei den im Folgenden beschriebenen Ausführungsbeispielen sind funktionell gleiche Teile mit gleichen Bezugszeichen wie vorstehend erläutert versehen, so dass insoweit hierauf verwiesen werden kann.

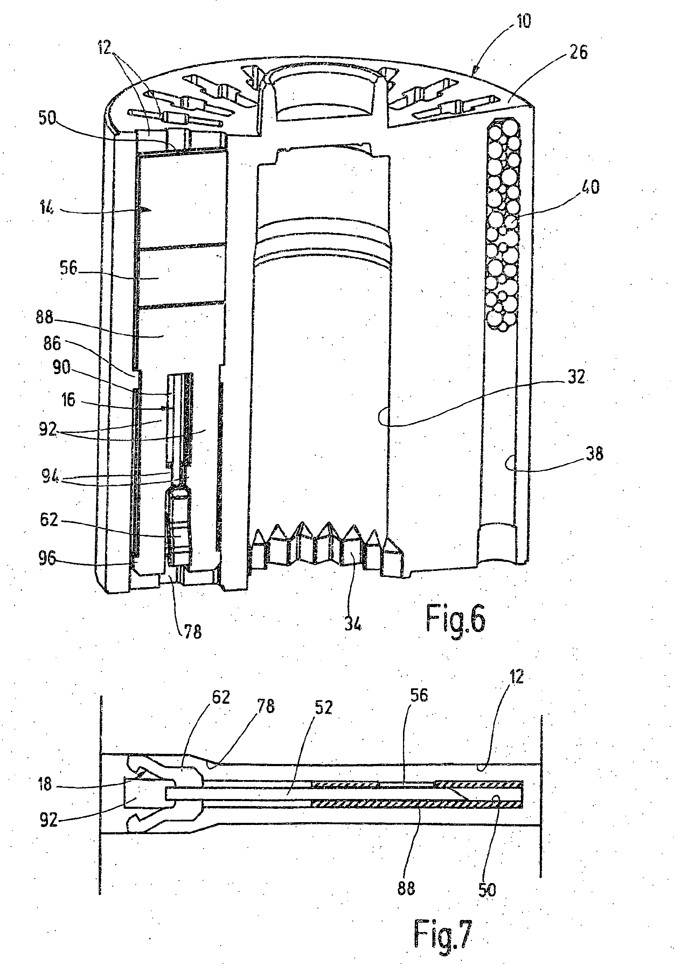

Das in Fig. 6 bis 10 gezeigte Ausführungsbeispiel unterscheidet sich vor allem dadurch, dass die Probenaufnahmeeinheit 14 als mehrlagiger Teststreifen 88 ausgebildet ist. Dieser ist zur Führung des Mitnehmers 62 der Stecheinheit 16 in seinem proximalen Abschnitt mit einer Ausstanzung 90 versehen, wobei die so gebildeten Streifenschenkel 92 mittige und endseitige Anschlagschultern 94, 96 aufweisen. Wie aus Fig. 7 ersichtlich, ist die Lanzette 52 in einer Zwischenlage des Teststreifens 88 in einem Längsspalt 50 geführt, welcher zugleich als kapillarer Fließpfad zu dem Testfeld 56 hin führt und durch Ausstanzen oder Einprägen gebildet sein kann. Aus herstellungstechnischen Gründen ist der Teststreifen nicht mit einem gesonderten Mitnehmer versehen, sondern wie nachstehend ausgeführt durch die wegabhängig wirksamen Anschlagschultern 94, 96 in seiner Bewegung gesteuert, während die Stecheinheit 16 über ihren zugehörigen Mitnehmer 62 die Steuerkulisse 78 der Führungskammer 12 abtastet.

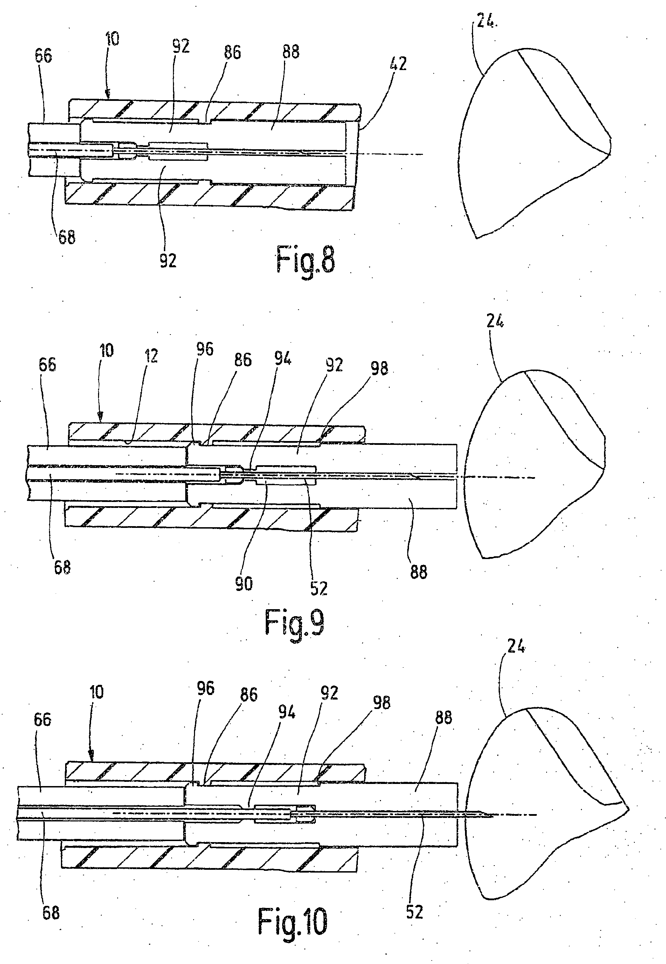

Der in Fig. 8 bis 10 gezeigte Ablauf der Probenaufnahme entspricht grundsätzlich der Sequenz nach Fig. 3 bis 5. Ein Unterschied besteht darin, dass der Außenstößel 66 nur auf Stoß gegen die zugewandte Stirnkante des Teststreifens 88 anliegt, um so das Durchstoßen der Siegelfolie 42 beim Vorschub zu erleichtern. In der in Fig. 9 gezeigten Anschlagstellung der endseitigen äußeren Anschlagschultern 96 gegenüber dem zweiseitig wirksamen Wandvorsprung 86 kann der Mitnehmer 62 die gegeneinander weisenden inneren Anschlagschultern 94 unter elastischer Aufspreizung der Streifenschenkel 92 passieren, wobei die äußeren Anschlagschultern 96 in nicht gezeigte Wandaussparungen eingreifen. Nach dem Einstechen stößt der in formschlüssiger Kopplung mit dem Innenstößel zurückgezogene Mitnehmer 62 rückseitig gegen die inneren Anschlagschultern 94 an, so dass auch der Teststreifen 88 in die Führungskammer 12 zurückgeholt wird. Die Freigabe des Innenstößels 68 erfolgt wiederum unter Aufspreizen der Streifenschenkel 92 in Anschlagstellung der Anschlagstufe 98 des Teststreifens 88 mit dem Wandvorsprung 86.

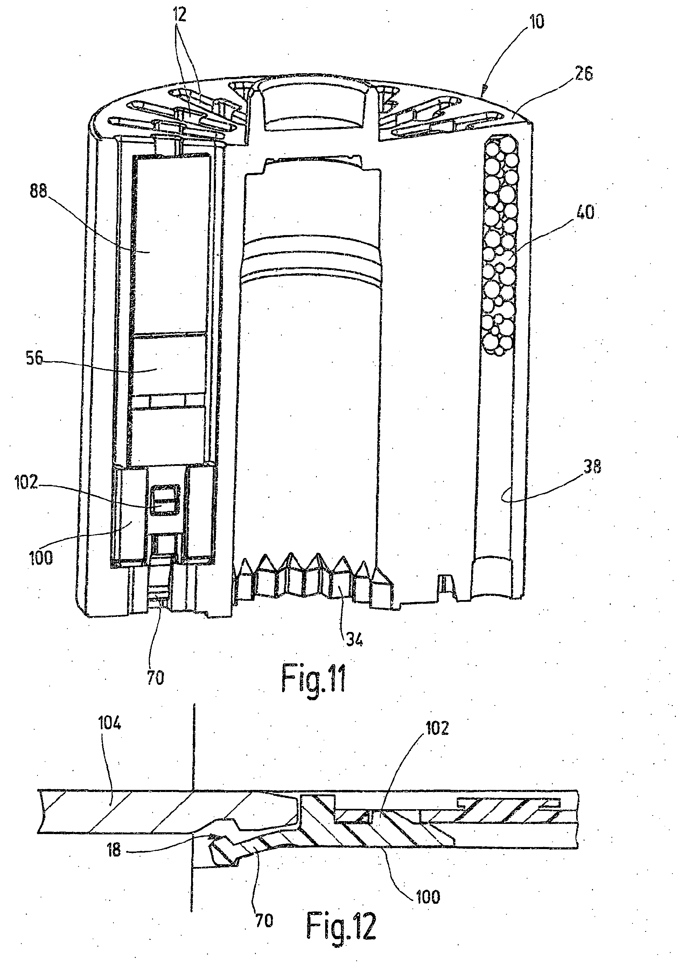

Bei der in Fig. 11 und 12 dargestellten Ausführungsform sind die Teststreifen 88 zur besseren Führung in einem Schlitten 100 gehalten, welcher in der jeweiligen Führungskammer 12 schubladenartig längsverschieblich ist. Der Schlitten 100 umgreift einen Endabschnitt des Teststreifens 88 und ist mit diesem über eine Rastnase 102 verbunden. Zur formschlüssigen Kopplung mit dem Einzelstößel 104 ist eine einzelne Halteklaue 70 des Schlittens 100 als Mitnehmer vorgesehen (Fig. 12). Anstelle einer in dem Teststreifen 88 integrierten Stecheinheit wird eine gesonderte, nicht gezeigte Stechhilfe eingesetzt.

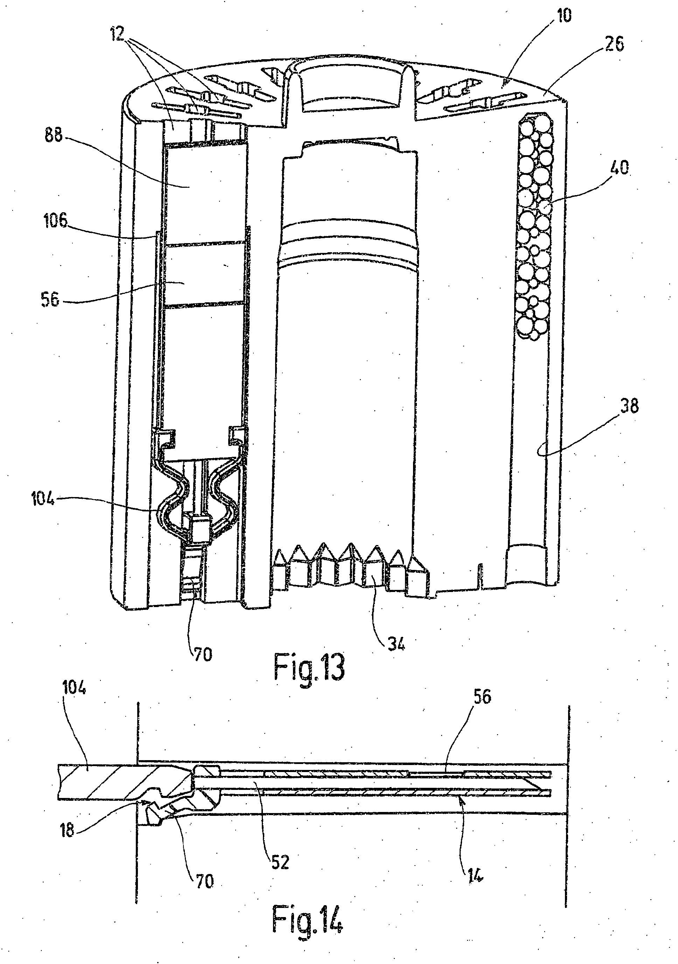

Bei dem in Fig. 13 und 14 gezeigten Ausführungsbeispiel ist eine in einen Teststreifen 88 integrierte Stecheinheit 16 entsprechend dem zuvor beschriebenen Schlitten 100 für eine Hin- und Rückbewegung über eine einzelne Halteklaue 70 als Mitnehmer mit einem einzelnen Antriebsstößel 104 formschlüssig verbindbar. Zur Bewegungsübertragung auf den Teststreifen 88 ist eine Federklammer 104 vorgesehen. Beim Vorschub wird gegenüber einer Wandstufe 106 eine Anschlagstellung des Folienstreifens 88 erreicht, in welcher die Lanzette 52 entgegen der Rückstellkraft der Federklammer 104 zum Einstechen weiter bewegt werden kann.

Wie auch bei dem Ausführungsbeispiel gemäß Fig. 7 wird der wirksame Querschnitt des Transportkanals 50 durch den Außendurchmesser der Lanzette 52 auf einen Ringspalt beschränkt. Da die Lanzette unter dem Einfluss der Schwerkraft nicht genau mittig liegt, ist der Spalt in dem von der Auflageseite abgewandten Bereich größer. Dieser Bereich kann durch eine entsprechende Formgebung zusätzlich erweitert werden, um den Blutfluss zu lenken und ihn über eine seitliche Auslassöffnung auf das Testfeld 56 zu lenken.

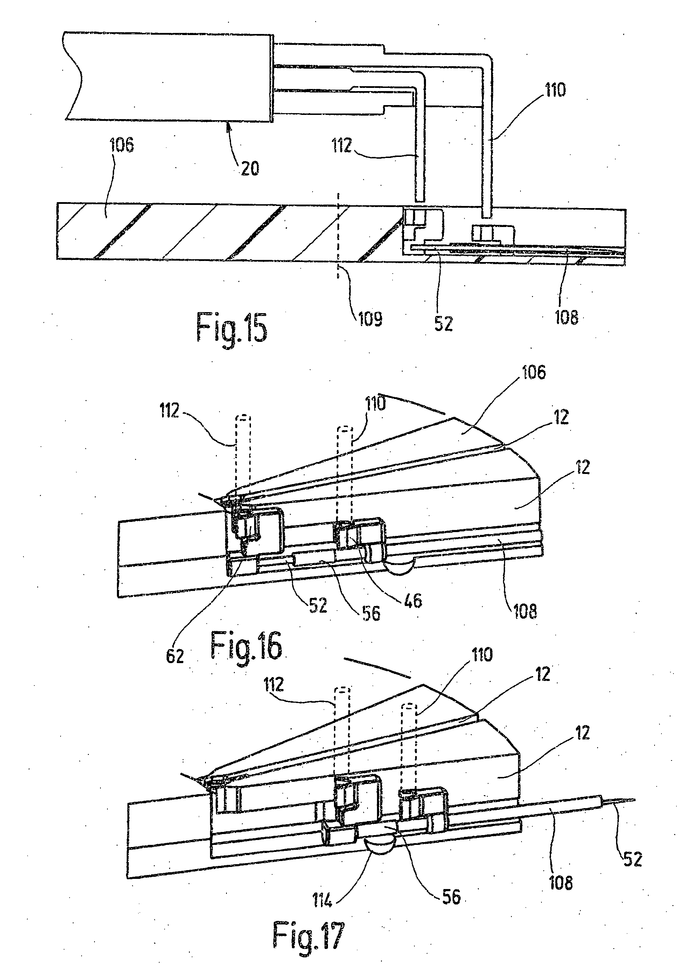

Die Ausführungsform gemäß Fig. 15 bis 17 umfasst ein flaches scheibenförmiges Magazin 106 als Behältnis 10, in welchem die Führungskammern 12 sternförmig radial verlaufen. Darin sind Kanülen 108 als Probenaufnahmeeinheiten 14 radial verschiebbar gelagert. Die Kanülen bzw. Probenröhrchen 108 bilden wiederum eine Führung für jeweils eine gesondert verschiebbare Lanzette 52. Die Antriebskopplung erfolgt über Mitnehmer 46, 62, die über achsparallel zu der Scheibenachse 109 in die stirnseitig randoffenen Führungskammern 12 eingreifende Winkelstößel 110, 112 der Antriebseinheit 20 nacheinander in die in Fig. 12 gezeigte vordere Vorschubstellung verschiebbar sind. Das aufgenommene Blut fließt über die Kanüle 108 axial zu dem hülsenförmigen Testfeld 56, dessen Ansprechen auf einen Analyten über das kalottenförmige Fenster 114 optisch erfasst werden kann.

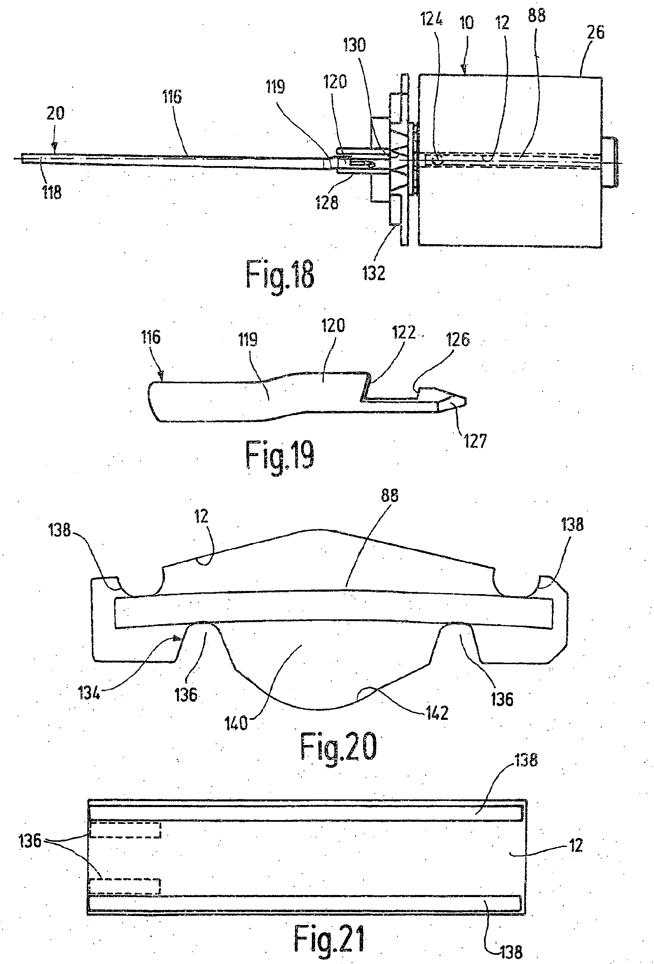

Bei dem in Fig. 18 bis 24 gezeigten Ausführungsbeispiel weist die Kopplungsvorrichtung 18 einen an die Teststreifen 88 in einem Trommelmagazin 26 ankoppelbaren Hakenstößel 116 auf, der an seinem proximalen Ende 118 für eine Hin- und Rückbewegung angetrieben ist. An seinem distalen Ende ist der Hakenstößel 116 mit einem über ein Kniestück 119 abgekröpften Hakenkopf 120 versehen. Dieser besitzt entsprechend Fig. 19 eine gegen das rückseitige Stirnende eines Teststreifens 88 anschlagende Schubflanke 122 für den Streifenvorschub und eine in eine Ausnehmung 124 des Teststreifens 88 einklinkbare Zugflanke 126 für die Streifenrückholung. Um die Anschlagstellung zu sichern und das Ausklinken zu erleichtern, sind die Flanken 122, 126 zu ihrer freien Randkante hin in Vorschubrichtung geneigt. Ein stirnseitig an dem Hakenkopf 120 angeformter und mit seiner Spitze in Vorschubrichtung weisender Dornfortsatz 127 ermöglicht ein leichtes Durchstechen einer die Führungskammer 12 dicht verschließenden Folie.

Der Hakenstößel 116 ist in einer Führungsbuchse 128 gelagert, welche über ein mit konischen Zentrieröffnungen 130 versehenes Trommel- bzw. Indexrad 132 an der Stirnseite des Trommelmagazins 26 auf die gewünschte Führungskammer ausrichtbar ist.

Wie am besten aus Fig. 20 und 21 ersichtlich, sind die Teststreifen 88 an ihrem proximalen Endabschnitt durch eine Klemmstruktur 134 in ihrer jeweiligen Führungskammer 12 lösbar gehalten. Zu diesem Zweck weist die Klemmstruktur 134 zwei in seitlichem Abstand voneinander befindliche, in die Führungskammer 12 vorspringende Klemmnocken 136 auf, die mit längs der Führungskammer 12 verlaufenden, gegenüberliegend nach außen versetzten Führungsrippen 138 zusammenwirken. Dabei wird der endseitig eingeklemmte Teststreifen 88 unter elastischer Biegeverformung quer ausgewölbt, so dass unterhalb des Teststreifens ein erweiterter Eingriffsquerschnitt 140 für den Hakenstößei 116 zur Verfügung steht.

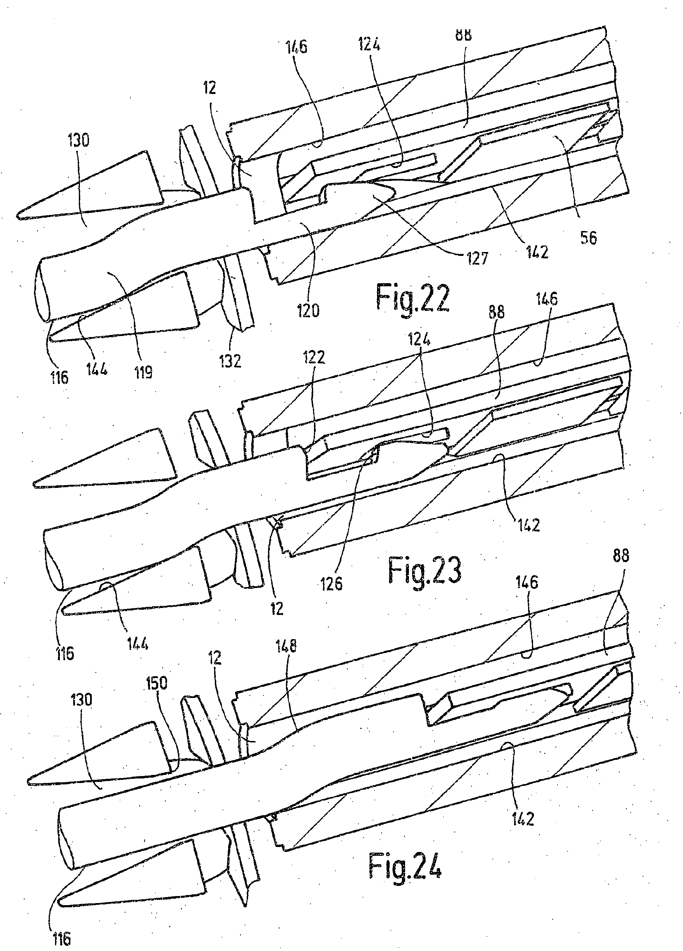

Fig. 22 bis 24 veranschaulicht die gesteuerte Führung des Hakenstößels 116 beim Ein- bzw. Auskoppelvorgang. Entsprechend der Stößelkröpfung ist der Führungskonus 130 gegenüber der Zentralachse der Führungskammer in Umfangsrichtung des Trommelrads 132 versetzt, wobei der Hakenkopf 120 zu dem Teststreifen 88 hin seitlich vorspringt. Beim Einstechen des Dornfortsatzes 127 bildet eine untere Wandung 142 der Führungskammer 12 eine Führungsfläche, bis das Kniestück 119 gegen die Schräge 144 des Führungskonus 130 anläuft. Hierdurch wird der Hakenkopf 120 unter einer Schwenkbewegung des Hakenstößels 116 angehoben, und die Zugflanke 126 hintergreift den Rand der Ausnehmung 124, während die Schubflanke 122 gegen das Streifenende anschlägt (Fig. 23). Beim weiteren Vorschub in der Anschlagstellung der Schubflanke 122 wird die Klemmkraft der Klemmstruktur 134 durch die höhere Antriebskraft der nicht gezeigten Antriebseinheit überwunden, wobei der Teststreifen 88 von den Klemmvorsprüngen 136 freikommt und schließlich in die Aufnahmestellung gelangt. Dabei erfolgt die Führung und Sicherung des gekröpften Hakenstößels zwischen den gegenüberliegenden Wandungen 142 und 146 der Führungskammer 12.

Bei der Rückwärtsbewegung fährt der Hakenstößel 116 mit der oberen Partie 148 des Kniestücks 119 gegen die obere Konuskante 150 des Führungskonus 130 und wird dadurch bis zum Auskoppeln in der Klemmstellung des Teststreifens 88 nach unten gedrückt. Durch die Neigung der Zugflanke 126 wird sichergestellt, dass die Sperrwirkung geringer ist als die maximale Zugkraft und der Hakenstößel 116 wieder aus der Ausnehmung 124 ausklinkt. Die Führungsbuchse 128 sorgt sowohl beim Vorschub als auch beim Rückholen dafür, dass der Hakenkopf 120 nicht am Trommelrad 132 einhakt. Der verbrauchte Teststreifen 88 wird lagerichtig klemmend zurückgehalten, und es kann auf die nächste Führungskammer in dem Magazin 26 weitergeschaltet werden.

## Patentansprüche

1. Vorrichtung zur Aufnahme einer Körperflüssigkeit für Analysezwecke, mit einem Behältnis (10) und mindestens einer mittels einer Antriebseinheit (20) aus einer Führungskammer (12) des Behältnisses ausschiebbaren und an einer Aufnahmestelle (22) im Bereich eines Körperteils mit der Körperflüssigkeit beaufschlagbaren, vorzugsweise für einen Einmaltest bestimmten Probenaufnahmeeinheit (14), und einer Kopplungsvorrichtung (18) zum Koppeln der Probenaufnahmeeinheit (14) mit der Antriebseinheit (20) für eine Hin- und Rückbewegung der Probenaufnahmeeinheit zwischen der Führungskammer (12) und der Aufnahmestelle (22), wobei in der Probenaufnahmeeinheit (14) eine Stecheinheit (16) zur Ausführung einer Stechbewegung gegen das die Körperflüssigkeit enthaltende Körperteil (24) integriert ist, **dadurch gekennzeichnet, dass** die Antriebseinheit (20) eine Steuereinrichtung zur Ablaufsteuerung besagter Bewegung von Probenaufnahmeeinheit (14) und Stecheinheit (16) aufweist.

2. Vorrichtung nach Anspruch 1, **dadurch gekennzeichnet, dass** die Stecheinheit (16) in einer Führung (50) der Probenaufnahmeeinheit (14) in Bewegungsrichtung der Probenaufnahmeeinheit (14) verschiebbar ist.

3. Vorrichtung nach Anspruch 1 oder 2, **dadurch gekennzeichnet, dass** die Kopplungsvorrichtung (18) vorzugsweise selbsttätig arbeitende Formschlussmittel (46,74;62,76) zum Herstellen und Lösen einer Formschlussverbindung zwischen Antriebseinheit (20) und Probenaufnahmeelnhelt (14) aufweist.

4. Vorrichtung nach einem der Ansprüche 1 bis 3, **dadurch gekennzeichnet, dass** die Antriebseinheit (20) und die Probenaufnahmeeinheit (14) in einer Ausgangsstellung voneinander getrennt sind.

5. Vorrichtung nach einem der Ansprüche 1 bis 4, **dadurch gekennzeichnet, dass** die Kopplungsvorrichtung (18) mindestens einen Mitnehmer (46,62) zur formschlüssigen Kopplung von Antriebseinheit (20) und Probenaufnahmeeinheit (14) aufweist.

6. Vorrichtung nach Anspruch 5, **dadurch gekennzeichnet, dass** der Mitnehmer (46,62) bei der Hin- und Rückbewegung wegabhängig zwischen einer Freigabestellung und einer Eingriffstellung bewegbar ist.

7. Vorrichtung einem Anspruch 5 oder 6, **dadurch gekennzeichnet, dass** die Kopplungsvorrichtung (18) eine bei der Hin- und Rückbewegung durch den Mitnehmer (46,62) abtastbare, insbesondere durch eine Anlaufschräge (77,80) gebildete Steuerkulisse (78) aufweist.

8. Vorrichtung nach einem der Ansprüche 5 bis 7, **dadurch gekennzeichnet, dass** der Mitnehmer (46,62) an einem proximalen Ende der Probenaufnahmeeinheit (14) angeordnet ist.

9. Vorrichtung nach einem der Ansprüche 5 bis 8, **dadurch gekennzeichnet, dass** der Mitnehmer (46,62) durch mindestens eine vorzugsweise unter Eigenspannung in eine Eingriffstellung einrückbare Halteklaue (70,72) gebildet ist.

10. Vorrichtung nach einem der Ansprüche 5 bis 9, **dadurch gekennzeichnet, dass** die Antriebseinheit (20) einen Stößel (64;66,68) aufweist, und dass der Mitnehmer (46,62) bei einem Axialvorschub des Stößels (64;66,68) selbsttätig mit einer Kopfpartie (74,76) des Stößels in Eingriff kommt.

11. Vorrichtung nach einem der Ansprüche 1 bis 10, **dadurch gekennzeichnet, dass** die Kopplungsvorrichtung (18) einen an der Probenaufnahmeeinheit (14) einhakbaren Hakenstößel (116) aufweist.

12. Vorrichtung nach einem der Ansprüche 1 bis 11, **dadurch gekennzeichnet, dass** die Stecheinheit (16) über einen zugeordneten Mitnehmer (62) der Kopplungsvorrichtung (18) für eine hin- und hergehende Stechbewegung formschlüssig mit der Antriebseinheit (20) koppelbar ist.

13. Vorrichtung nach einem der Ansprüche 1 bis 12, **dadurch gekennzeichnet, dass** die Antriebseinheit (20) einen durch einen Außenstößel (66) und einen darin längsverschiebbaren Innenstößel (68) gebildeten Doppelstößel (64) zum gesonderten Antrieb von Probenaufnahmeeinheit (14) und Stecheinheit (16) aufweist.

## Claims

1. Device for receiving a body fluid for analytical purposes comprising a container (10) and at least one sample receiving unit (14) preferably for a single-use test that can be pushed out of a guide chamber (12) of the container by means of a drive unit (20) and to which the body fluid can be applied at a receiving site (22) in the area of a body part, and a coupling device (18) to couple the sample receiving unit (14) to the drive unit (20) for a forwards and backwards movement of the sample receiving unit between the guide chamber (12) and the receiving site (22), wherein a lancing unit (16) is integrated in the sample receiving unit (14) to carry out a lancing movement towards the body part (24) containing the body fluid, **characterized in that** the drive unit (20) has a control device for sequence control of said movement of the sample receiving unit (14) and lancing unit (16).

2. Device according to claim 1, **characterized in that** the lancing unit (16) is slidable in a guide (50) of the sample receiving unit (14) in direction of movement of the sample receiving unit (14).

3. Device according to claim 1 or 2, **characterized in that** the coupling device (18) has preferably automatically operating form-fitting means (46, 74; 62, 76) to make and disengage a form-fitting connection between the drive unit (20) and sample receiving unit (14).

4. Device according to one of the claims 1 to 3, **characterized in that** the drive unit (20) and the sample receiving unit (14) are separated from one another in an initial position.

5. Device according to one of the claims 1 to 4, **characterized in that** the coupling device (18) has at least one engaging means (46, 62) to couple the drive unit (20) and sample receiving unit (14) in a form-fitting manner.

6. Device according to claim 5, **characterized in that** the engaging means (46, 62) can be moved in a distance-dependent manner between a release position and an engaging position during the forwards and backwards movement.

7. Device according to claim 5 or 6, **characterized in that** the coupling device (18) has a sliding guide block (78) and in particular one that is formed by an inclined bevel (77, 80) that can be tracked by the engaging means (46, 62) during the forwards and backwards movement.

8. Device according to one of the claims 5 to 7, **characterized in that** the engaging means (46, 62) is located at a proximal end of the sample receiving unit (14).

9. Device according to one of the claims 5 to 8, **characterized in that** the engaging means (46, 62) is formed by at least one holding claw (70, 72) that can be shifted into an engaging position preferably under its own tension.

10. Device according to one of the claims 5 to 9, **characterized in that** the drive unit (20) has a plunger (64; 66, 68) and that the engaging means (46, 62) automatically engages a head member (74, 76) of the plunger when the plunger (64; 66, 68) is advanced axially.

11. Device according to one of the claims 1 to 10, **characterized in that** the coupling device (18) comprises a hooked plunger (116) that can be hooked onto the sample receiving unit (14).

12. Device according to one of the claims 1 to 11, **characterized in that** the lancing unit (16) can be coupled in a form-fitting manner to the drive unit (20) by means of an associated engaging means (62) of the coupling device (18) for a reciprocating lancing movement.

13. Device according to one of the claims 1 to 12, **characterized in that** the drive unit (20) has a double plunger (64) formed by an outer plunger (66) and an inner plunger (68) that can be longitudinally moved therein to separately drive the sample receiving unit (14) and lancing unit (16).

## Revendications

1. Dispositif destiné à une réception d'un liquide corporel à des fins d'analyse, comprenant un récipient (10) et au moins une unité de réception d'échantillon (14) conçue de manière préférée pour un test unique, pouvant être poussée hors d'un compartiment de guidage (12) du récipient au moyen d'une unité d'entraînement (20) et pouvant être alimentée en liquide corporel au niveau d'un emplacement de réception (22) dans le secteur d'une partie corporelle, et un dispositif de couplage (18) destiné à coupler l'unité de réception d'échantillon (14) avec l'unité d'entraînement (20) pour un mouvement en avant et en arrière de l'unité de réception d'échantillon entre le compartiment de guidage (12) et l'emplacement de réception (22), une unité de piquage (16) destinée à une réalisation d'un mouvement de piquage contre la partie corporelle (24) contenant le liquide corporel étant intégrée dans l'unité de réception d'échantillon (14), **caractérisé en ce que** l'unité d'entraînement (20) présente un appareil de commande destiné à commander un déroulement dudit mouvement de l'unité de réception d'échantillon (14) et de l'unité de piquage (16).

2. Dispositif selon la revendication 1, **caractérisé en ce que** l'unité de piquage (16) peut être déplacée dans une direction de déplacement de l'unité de réception d'échantillon (14) au sein d'un guidage (50) de l'unité de réception d'échantillon (14).

3. Dispositif selon la revendication 1 ou 2, **caractérisé en ce que** le dispositif de couplage (18) présente de manière préférée un moyen de fermeture par complémentarité de forme (46, 74 ; 62, 76) fonctionnant de manière préférée de manière automatique et destiné à établir et défaire une liaison par complémentarité de forme entre l'unité d'entraînement (20) et l'unité de réception d'échantillon (14).

4. Dispositif selon l'une quelconque des revendications 1 à 3, **caractérisé en ce que** l'unité d'entraînement (20) et l'unité de réception d'échantillon (14) sont séparées l'une de l'autre dans une position initiale.

5. Dispositif selon l'une quelconque des revendications 1 à 4, **caractérisé en ce que** le dispositif de couplage (18) présente au moins un toc d'entraînement (46, 62) destiné à un couplage par complémentarité de forme de l'unité d'entraînement (20) et de l'unité de réception d'échantillon (14).

6. Dispositif selon la revendication 5, **caractérisé en ce que** le toc d'entraînement (46, 62) peut être déplacé entre une position de libération et une position de mise en prise en fonction de sa course lors du mouvement vers l'avant et vers l'arrière.

7. Dispositif selon la revendication 5 ou 6, **caractérisé en ce que** le dispositif de couplage (18) présente une coulisse de commande (78) pouvant être explorée par le toc d'entraînement (46, 62) lors du mouvement en avant et en arrière, et en particulier formée d'un chanfrein de démarrage (77, 80).

8. Dispositif selon l'une quelconque des revendications 5 à 7, **caractérisé en ce que** le toc d'entraînement (46, 62) est agencé sur une extrémité proximale de l'unité de réception d'échantillon (14).

9. Procédé selon l'une quelconque des revendications 5 à 8, **caractérisé en ce que** le toc d'entraînement (46, 62) est formé d'au moins une griffe de retenue (70, 72) pouvant être enclenchée dans une position de mise en prise de manière préférée sous l'effet d'une contrainte interne.

10. Dispositif selon l'une quelconque des revendications 5 à 9, **caractérisé en ce que** l'unité d'entraînement (20) présente un poussoir (64 ; 66, 68), et le toc d'entraînement (46, 62) vient en prise de manière automatique avec une partie tête (74, 76) du poussoir lors d'un avancement axial du poussoir (64 ; 66, 68).

11. Dispositif selon l'une quelconque des revendications 1 à 10, **caractérisé en ce que** le dispositif de couplage (18) présente un poussoir à crochet (116) pouvant être accroché à l'unité de réception d'échantillon (14).

12. Dispositif selon l'une quelconque des revendications 1 à 11, **caractérisé en ce que** l'unité de piquage (16) peut être couplée par complémentarité de forme avec l'unité d'entraînement (20) par l'intermédiaire d'un toc d'entraînement (62) associé du dispositif de couplage (18) en vue d'un mouvement de piquage en avant et en arrière.

13. Dispositif selon l'une quelconque des revendications 1 à 12, **caractérisé en ce que** l'unité d'entraînement (20) présente un poussoir double (64) formé d'un poussoir externe (66) et d'un poussoir interne (68) pouvant être déplacé de manière longitudinale à l'intérieur dudit poussoir externe en vue d'un entraînement séparé de l'unité de réception d'échantillon (14) et de l'unité de piquage (16).
